# FASCICULE DE BREVET EUROPEEN

(11) **EP 1 358 159 B1**
(45) Date de publication et mention de la délivrance du brevet: **22.07.2009**
(21) Numéro de dépôt: 02701374.7
(22) Date de dépôt: 01.02.2002
(51) Int. Cl.: C07D 213/73, A61K 31/44, A61P 21/00, A61P 21/04

(54) **TARTRATE ET PHOSPHATE DE 3,4-DIAMINOPYRIDINE, COMPOSITIONS PHARMACEUTIQUES ET UTILISATIONS**
TARTRAT- UND PHOSPHATSALZ VON 3,4-DIAMINOPYRIDIN, PHARMAZEUTISCHE ZUSAMMENSETZUNG UND DEREN VERWENDUNG
3,4-DIAMINOPYRIDINE TARTRATE AND PHOSPHATE, PHARMACEUTICAL COMPOSITIONS AND USES THEREOF

(30) Priorité: 05.02.2001 FR 0101495
(43) Date de publication de la demande: 05.11.2003
(73) Titulaire: ASSISTANCE PUBLIQUE, HOPITAUX DE PARIS, 75004 Paris (FR); Guyon, Francois, 75005 Paris (FR); Pradeau, Dominique, 94130 Nogen-sur-Marne (FR); Le Hoang, My Dung, 75011 Paris (FR); Houri, Jean-Jacques, 94170 Le Perreux sur Marne (FR)
(72) Inventeur: GUYON, François, F-75005 Paris (FR); PRADEAU, Dominique, F-94130 Nogent-sur-Marne (FR); LE HOANG, My, Dung, F-75011 Paris (FR); HOURI, Jean-Jacques, F-94170 Le Perreux sur Marne (FR)
(74) Mandataire: Goulard, Sophie
(86) Numéro de dépôt international: PCT/FR2002/000387
(87) Numéro de publication internationale: WO 2002/062760

(56) Documents cités:
- EP-A- 0 159 112
- WO-A-00/47210
- WO-A-97/22339
- WO-A-97/34624
- DATABASE CA [en ligne] CHEMICAL ABSTRACTS SERVICE, COLUMBUS, OHIO, US; LIPPMANN, EBERHARD ET AL: "Quinoxalines. VI. Reactions of.alpha.-substituted carbonyl compounds with 3,4-diaminopyridines" retrieved from STN Database accession no. 76:14489 XP002180231 & Z. CHEM. (1971), 11(9), 343,
- SANDERS, DONALD B.: "3,4-Diaminopyridine (DAP) in the treatment of Lambert-Eaton myasthenic syndrome (LEMS)" ANN. N. Y. ACAD. SCI. (1998), 841(MYASTHENIA GRAVIS AND RELATED DISEASES), 811-816, XP001030622
- MOLGO, J. ET AL: "3,4-Diaminopyridine, an orphan drug, in the symptomatic treatment of Lambert-Eaton myasthenic syndrome" PFLUEGERS ARCH. (1996), 431(6, SUPPL. 2, INTERNATIONAL MEETING, "LIFE SCIENCES 1995"), R295-R296, XP001030624
- KÖNIGS ET AL: "Reaktionen von alpha-substituierten Carbonylverbindungen mit 3,4-Diaminopyridin" Z.CHEM, vol. 11, no. 9, 1971, pages 343-344, XP008074480

## Description

La présente invention est relative au phosphate de 3,4-diaminopyridine, aux compositions pharmaceutiques contenant du tartrate ou du phosphate de 3,4-diaminopyridine, ainsi qu'à leurs utilisations.

L'utilisation de dérivés de pyridine dans le domaine pharmaceutique a largement été décrite pour diverses applications et notamment pour le traitement de la myasthénie ou des syndromes myasthéniques.

Le fait commun à la myasthénie et aux différents syndromes myasthéniques actuellement connus est la fatigabilité à l'effort. On peut distinguer d'une part la myasthénie (*myasthenia gravis :* MG) qui est une maladie auto-immune, et d'autre part les syndromes myasthéniques qui sont un assemblage d'états forts disparates :
- le syndrome myasthénique de Lambert-Eaton (SMLE) qui peut-être considéré comme d'origine auto-immune mais qui n'est pas nécessairement un syndrome paranéoplasique,
- la myasthénie congénitale,
- les syndromes myasthéniques d'origine médicamenteuse ou toxique.

Tous ces états sont consécutifs à l'atteinte de la jonction neuromusculaire à différents niveaux.

Il a par exemple été décrit que certains dérivés de pyridine tels que la 4-aminopyridine (4-AP) et la 3,4-diaminopyridine (3,4-DAP) peuvent être utilisés pour le traitement de la myasthénie et des syndromes myasthéniques car ils améliorent la transmission neuromusculaire en augmentant l'entrée du calcium cellulaire, ce qui favorise la libération d'acétylcholine au niveau des terminaisons nerveuses (Murray N.M. et al., Neurology, 1981, 31, 265-271).

L'article de McEvoy K.M. et al. (N. Engl. J. Med., 1989, 321, 1567-1571) indique à ce propos que la 3,4-DAP peut être efficacement utilisée pour le traitement du syndrome myasthénique de Lambert-Eaton.

D'autre part, des études sur animaux ont suggéré que, dans cette application particulière, la 3,4-DAP serait plus efficace et aurait moins d'effets secondaires que la 4-AP (Lemeighan M. et al., Brain Res., 1984, 304, 166-169 et Paskov D.S et al., Eksp. Khir. Anestexiol., 1973, 18, 48-52.

D'autre part, la capacité de la 3,4-DAP à augmenter la libération d'acétylcholine au niveau des terminaisons nerveuses permet également d'envisager son utilisation pour améliorer les fonctions cognitives lors du vieillissement (US 4,386,095). Il est également connu de la demande internationale WO 97/22339, que la 3,4-DAP permet d'inhiber l'activité de l'acétylcholine estérase et peut ainsi être utilisée dans le traitement des désordres respiratoires durant le sommeil.

Il a également déjà été proposé d'utiliser la 3,4-DAP pour le traitement symptomatique de la fatigue liée à une pathologie neurologique, telle que par exemple la sclérose en plaque (Bever et al., Annals of Neurology, 1990, 27, 421-427 et Sheean et al., Brain, 1998, 121, 967-975).

Enfin, l'utilisation d'aminopyridines, et en particulier de la 3,4-DAP a déjà été proposée, notamment dans le brevet US 5,952,357, pour le traitement des maladies affectant les cellules neuronales motrices, telles que la poliomyélite infectieuse aiguë et ses suites, le syndrome de Creutzfeld-Jacob, certains désordres toxiques et nutritionnels tels que ceux liés à une carence en vitamine B12, la dégénération des neurones moteurs suite à une exposition à certains composés tels que l'aluminium, les maladies dégénératives telles que la sclérose latérale amyotrophique, la sclérose latérale primaire, la démence pré-sénile avec atteinte des neurones moteurs, les atrophies musculaires spinales, l'atrophie olivopontocérébelleuse, la maladie de Joseph, la maladie de Parkinson, la chorée de Huntington ou encore la maladie de Pick.

Cependant, bien que l'efficacité thérapeutique de la 3,4-DAP ait été reconnue, l'utilisation de ce composé, sous forme de base libre, dans des formes médicamenteuses conduit à des périodes de validité du médicament correspondant, conservé dans les conditions de stabilité conformes aux recommandations internationales *(*International Conference on Harmonization, Draft Guideline on Stability testing of New Drug and Product, novembre 1999, CPMP/ICH/2736/99), très réduites. Ainsi, il n'est actuellement pas possible d'envisager une commercialisation de la 3,4-DAP.

C'est afin de remédier à ces problèmes que, de façon surprenante, les Inventeurs ont mis au point ce qui fait l'objet de l'invention.

Les Inventeurs se sont donc fixés pour objectif de pourvoir à des composés ayant des propriétés thérapeutiques au moins équivalentes à celles de la 3,4-DAP sous forme de base libre, mais présentant une stabilité améliorée au cours du temps, notamment après leur incorporation dans une forme médicamenteuse et ont découvert que certains sels de la 3,4-DAP permettent de répondre à cet objectif.

La présente invention a donc pour objet le phosphate de 3,4-diaminopyridine, ainsi qu'une composition pharmaceutique renfermant, à titre de principe actif, du tartrate ou du phosphate de 3,4-diaminopyridine, et éventuellement au moins un véhicule pharmaceutiquement acceptable.

Les Inventeurs ont en effet démontré que le tartrate et le phosphate de 3,4-DAP présentent une très grande stabilité dans les conditions de conservation conformes aux recommandations internationales alors que, dans ces mêmes conditions, la 3,4-DAP est instable.

Ces sels particuliers de la 3,4-DAP présentent également une plus grande solubilité dans l'eau, facilitant ainsi leur formulation.

Ces composés se présentent sous la forme d'une poudre cristalline blanche très soluble dans l'eau.

Ces composés peuvent être préparés selon un procédé de préparation consistant à faire réagir de la 3,4-DAP avec de l'acide tartrique ou de l'acide phosphorique, afin d'obtenir le tartrate ou le phosphate de 3,4-DAP correspondant.

La composition pharmaceutique conforme à l'invention présente les propriétés de pouvoir être utilisée dans les mêmes indications que la 3,4-DAP comme par exemple pour le traitement du botulisme, de la myasthénie, des syndromes myasthéniques ainsi que de la fatigue liée à une pathologie neurologique telle que par exemple la sclérose en plaque ou la sclérose latérale amyotrophique.

La composition pharmaceutique conforme à l'invention peut être administrée par voie orale à raison de 3 à 4 prises par jour en utilisation chronique ou par voie injectable.

La composition pharmaceutique conforme à l'invention peut donc se présenter sous des formes diverses telles que sous la forme de gélules, de capsules, de comprimés, de suspensions buvables, de tablettes, de solutions injectables ou sous toute autre forme appropriée au mode d'administration par voie orale ou injectable.

La quantité de tartrate ou de phosphate de 3,4-DAP présente dans la composition pharmaceutique conforme à l'invention correspond de préférence à des doses unitaires comprises entre 5 mg et 20 mg, exprimées en poids de 3,4-DAP sous forme de base libre.

La nature du véhicule pharmaceutiquement acceptable, éventuellement présent au sein de la composition pharmaceutique conforme à l'invention, variera bien entendu en fonction du mode d'administration et de la présentation galénique de ladite composition.

Le véhicule pharmaceutique est généralement constitué d'un ou de plusieurs excipients classiquement utilisés pour la préparation de compositions pharmaceutiques, tels que des anti-agglomérants, des anti-oxydants, des colorants, des vitamines, des sels minéraux, des agents modifiants du goût, des agents de lissage, de montage, d'isolation, leurs mélanges, et de manière générale, tout excipient classiquement utilisé dans l'industrie pharmaceutique.

Bien entendu, l'homme de l'art veillera à cette occasion à ce que le ou les additifs éventuellement utilisés soient compatibles avec les propriétés intrinsèque attachées à la composition pharmaceutique conforme à l'invention.

La composition pharmaceutique conforme à l'invention peut en outre renfermer un ou plusieurs principes actifs additionnels.

Enfin, l'invention a pour objet l'utilisation du tartrate ou du phosphate de 3,4-diaminopyridine pour la préparation d'une composition pharmaceutique destinée au traitement du botulisme, de la myasthénie, des syndromes myasthéniques ou de la fatigue liée à une pathologie neurologique telle que la sclérose en plaque ou la sclérose latérale amyotrophique.

Outre les dispositions qui précèdent, l'invention comprend encore d'autres dispositions qui ressortiront de la description qui va suivre, qui se réfère à deux exemples de préparation du tartrate et du phosphate de 3,4-DAP ainsi qu'à un exemple concernant l'étude de la stabilité du tartrate de 3,4-DAP.

Il doit être bien entendu toutefois que ces exemples sont donnés uniquement à titre d'illustration de l'objet de l'invention, dont ils ne constituent en aucune manière une limitation.

### EXEMPLE 1 : PRÉPARATION DU TARTRATE DE 3,4-DIAMINOPYRIDINE

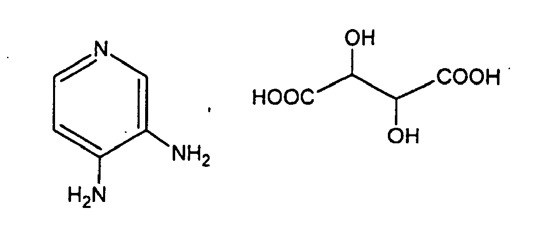

Dans un réacteur, sont introduits 100 parts de 3,4-DAP (Aldrich) préalablement purifiée et 300 parts d'eau distillée. Le mélange est porté à température d'ébullition, sous agitation.

Séparément, on dissout 135,5 parts d'acide L-tartrique dans 200 parts d'eau distillée. Lentement, la solution d'acide L-tartrique est introduite dans la solution bouillante de 3,4-DAP. On laisse ensuite le mélange réactionnel au reflux pendant 15 minutes, puis on le refroidit à 70°C.

1 part de charbon actif en poudre est alors ajoutée et on poursuit l'agitation pendant 15 minutes. Ensuite la séparation du charbon est réalisée par filtration.

La solution incolore est ensuite refroidie progressivement à 40°C et maintenue à cette température pendant 12 heures sous agitation. Après essorage et lavage avec 50 parts d'éthanol absolu, le produit est séché à 60°C sous vide jusqu'à poids constant. On obtient alors 164 part de tartrate de 3,4-DAP dont le point de fusion est compris entre 178 et 180°C. Le tartrate de 3,4-DAP peut ensuite être à nouveau purifié par une cristallisation dans l'eau.

L'analyse élémentaire du produit ainsi obtenu a été réalisée sur un appareil PERKIN ELMER CHN 4000. L'échantillon de produit est pesé sur une balance dont la précision est de 10⁻⁴ mg, le pourcentage d'oxygène a été calculé par différence.

L'analyse élémentaire du produit obtenu, conforme à celle du produit attendu, est la suivante :

| **%** | **C** | **H** | **N** | **O** |
|---|---|---|---|---|
| **Calculé** | 41,70 | 5,06 | 16,21 | 37,03 |
| **Trouvé** | 41,71 | 5,07 | 16,22 | 37,00 (*) |

| | | | | |
|---|---|---|---|---|
| (*) calculé par différence : [100-(C% + H% + N%)] | | | | |

### EXEMPLE 2 : PRÉPARATION DU PHOSPHATE DE 3,4-DIAMINOPYRIDINE

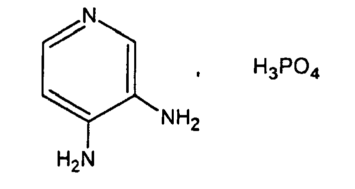

### Etape 1) : synthèse du phosphate de 3,4-DAP

Dans un réacteur, sont introduits 90 parts de 3,4-DAP (Aldrich) préalablement purifiée et 1800 parts d'eau distillée. Le mélange est porté à une température de 75°C, sous agitation. On observe une dissolution totale.

Ensuite, 191 parts d'acide phosphorique à 85% sont introduites lentement dans la solution de 3,4-DAP. Après l'addition de l'acide phosphorique, le mélange réactionnel est maintenu pendant encore 15 minutes à une température de 80°C, puis refroidi à 35°C.

Le mélange réactionnel est alors maintenu pendant 4 heures, sous agitation, à une température comprise entre 30 et 35°C.

Le précipité formé est essoré et lavé avec 100 parts d'eau distillée puis avec 100 parts d'éthanol absolu. Après séchage sous vide à 60°C jusqu'à poids contant, on obtient 160 parts de phosphate de 3,4-DAP brut sous la forme d'une poudre blanche dont le point de fusion est compris entre 225 et 227°C.

### Etape 2) Purification du phosphate de 3,4-DAP brut

Dans un réacteur, on introduit 160 parts de phosphate de 3,4-DAP brut obtenu ci-dessus à l'étape (1), 640 parts d'éthanol absolu et 715 parts d'eau distillée. Le mélange est chauffé, sous agitation, à une température de 80°C. A cette température, la dissolution est totale.

Le mélange réactionnel est ensuite refroidi progressivement à une température de 4°C et maintenu à cette température pendant 12 heures sous agitation.

Après essorage et lavage avec 100 parts d'éthanol absolu, on obtient 180 parts de produit humide. Le produit est ensuite séché à 60°C sous vide jusqu'à poids constant. On obtient alors 133 parts de phosphate de 3,4-DAP dont le point de fusion est de 229°C

L'analyse élémentaire du produit obtenu a été effectuée dans les mêmes conditions que celles décrites ci-dessus à l'exemple 1.

L'analyse élémentaire du produit obtenu, conforme à celle du produit attendu, est la suivante :

| **%** | **C** | **H** | **N** | **P** | **O** |
|---|---|---|---|---|---|
| **Calculé** | 28,99 | 4,83 | 20,29 | 14,97 | 30,92 |
| **Trouvé** | 29,05 | 4,93 | 20,23 | non déterminé | non déterminé |

### EXEMPLE 3 : ÉTUDE DE STABILITÉ DU TARTRATE DE 3,4-DIAMINOPYRIDINE

Du tartrate de 3,4-DAP, tel que préparé ci-dessus à l'exemple 1 a été introduit dans des gélules en gélatine de taille n°3, à raison de 10 mg (exprimés en poids de 3,4-DAP sous forme de base libre) par gélule (Gélules A).

De la même façon et à titre comparatif, des gélules en gélatine de taille n°3 renfermant 10 mg de 3,4-DAP par gélule ont été préparées (Gélules B).

Une étude de stabilité du tartrate de 3,4-DAP et de la 3,4-DAP, introduits respectivement dans les gélules A et B, a ensuite été réalisée.

Cette étude a été effectuée dans les conditions préconisées par les recommandations internationales pour l'étude de la stabilité des principes actifs (International Conference on Harmonization, Draft Guideline on Stability testing of New Drug and Product, novembre 1999, CPMP/ICH/2736/99).

Les résultats obtenus figurent dans les tableaux I et II ci-après :

**TABLEAU I**

| | **T = 0** | | **T = 3 mois (conservation à 25°C, humidité relative de 60)** | |
|---|---|---|---|---|
| | **Aspect de la poudre** | **Teneur en principe actif (*)** | **Aspect de la poudre** | **Teneur en principe actif** |
| **Gélule A (tartrate de 3,4-DAP)** | blanche cristalline | 95,6 | blanche cristalline | 97,1 |
| **Gélule B (3,4-DAP)** | blanche cristalline | 98,5 | Jaune (produit de dégradation) | non déterminée |

| | | | | |
|---|---|---|---|---|
| (*) pourcentage de la valeur cible | | | | |

**TABLEAU II**

| **Conditions de conservation :** - Température : 40°C - Humidité relative : 75 % | | **Gélule A (tartrate de 3,4-DAP)** | **Gélule B (3,4-DAP)** |
|---|---|---|---|
| **T** = **0** | Aspect de la poudre | Blanche cristalline | Blanche cristalline |
| | Teneur en principe actif (*) | **95.6** | 98.5 |
| **T** = **3 mois** | Aspect de la poudre | Blanche cristalline | Jaune (produit de dégradation) |
| | Teneur en principe actif (*) | **96,1** | Non déterminé |
| **T** = **6 mois** | Aspect de la poudre | Poudre de couleur crème | Jaune (produit de dégradation) |
| | Teneur en principe actif (*) | **97,9** | Non déterminé |

| | | | |
|---|---|---|---|
| (*) pourcentage de la valeur cible | | | |

Ces résultats montrent que le tartrate de 3,4-DAP, formulé en gélules, présente une grande stabilité alors que la 3,4-DAP est instable.

## Revendications

1. Phosphate de 3,4-diaminopyridine.

2. Composition pharmaceutique **caractérisée par le fait qu'**elle renferme, à titre de principe actif, du tartrate ou du phosphate de 3,4-diaminopyridine, et éventuellement au moins un véhicule pharmaceutiquement acceptable.

3. Composition pharmaceutique selon la revendication 2, **caractérisée par le fait qu'**elle est destinée à être administrée par voie orale ou injectable et qu'elle se présente sous la forme de gélules, de capsules, de comprimés, de suspensions buvables, de tablettes ou de solutions injectables.

4. Composition selon la revendication 2 ou 3, **caractérisée par le fait que** la quantité de tartrate ou de phosphate de 3,4-DAP présente correspond à des doses unitaires comprises entre 5 mg et 20 mg, exprimée en poids de 3,4-DAP sous forme de base libre.

5. Composition selon l'une quelconque des revendications 2 à 4, **caractérisée par le fait que** le véhicule pharmaceutiquement acceptable est choisi parmi les anti-agglomérants, les anti-oxydants, les colorants, les vitamines, les sels minéraux, les agents modifiants du goût, les agents de lissage, de montage, d'isolation et leurs mélanges.

6. Composition selon l'une quelconque des revendications 2 à 5, **caractérisée par le fait qu'**elle renferme un ou plusieurs principe actif additionnel.

7. Utilisation du tartrate ou du phosphate de 3,4-diaminopyridine pour la préparation d'une composition pharmaceutique destinée au traitement du botulisme, de la myasthénie, des syndromes myasthéniques ou de la fatigue liée à une pathologie neurologique telle que la sclérose en plaque ou la sclérose latérale amyotrophique.

## Claims

1. 3,4-Diaminopyridine phosphate.

2. A pharmaceutical composition **characterised by** the fact that it comprises, as active ingredient, 3,4-diaminopyridine tartrate or phosphate, and optionally at least one pharmaceutically acceptable vehicle.

3. A pharmaceutical composition according to claim 2, **characterised by** the fact that it is intended to be administered by oral or injectable route and that it assumes the form of hard capsules, soft capsules, tablets, drinkable suspensions, lozenges or solutions for injection.

4. A composition according to claim 2 or claim 3, **characterised by** the fact that the quantity of 3,4-DAP tartrate or phosphate present corresponds to unit doses of between 5 mg and 20 mg, stated by weight of 3,4-DAP in free base form.

5. A composition according to any one of claims 2 to 4, **characterised by** the fact that the pharmaceutically acceptable vehicle is selected from among antiagglomerants, antioxidants, colorants, vitamins, mineral salts, flavour modifiers, smoothing, assembly, isolation agents and mixtures thereof.

6. A composition according to any one of claims 2 to 5, **characterised by** the fact that it comprises one or more additional active ingredient(s).

7. Use of 3,4-diaminopyridine tartrate or phosphate for preparing a pharmaceutical composition intended for the treatment of botulism, myasthenia, myasthenic syndromes or fatigue associated with a neurological disease, such as disseminated sclerosis or amyotrophic lateral sclerosis.

## Patentansprüche

1. 3,4-Diaminopyridin-Phosphat.

2. Pharmazeutische Zusammensetzung, **dadurch gekennzeichnet, dass** sie als Wirkstoff 3,4-Diaminopyridin-Tartrat oder -Phosphat und gegebenenfalls wenigstens ein pharmazeutisch verträgliches Vehikel umfasst.

3. Pharmazeutische Zusammensetzung gemäß Anspruch 2, **dadurch gekennzeichnet , dass** sie dazu bestimmt ist, auf oralem Weg oder als Injektion verabreicht zu werden und dass sie sich in Form von Gelatinekapseln, Kapseln, Tabletten, trinkbaren Suspensionen, Pastillen oder injizierbaren Lösungen präsentiert.

4. Zusammensetzung gemäß Anspruch 2 oder 3, **dadurch gekennzeichnet, dass** die Menge an 3,4-DAP-Tartrat oder -Phosphat, die vorliegt, Einzeldosen zwischen 5 mg und 20 mg, ausgedrückt als Gewicht von 3,4-DAP in Form der freien Base, entspricht.

5. Zusammensetzung gemäß einem der Ansprüche 2 bis 4, **dadurch gekennzeichnet , dass** das pharmazeutisch verträgliche Vehikel aus Agglutinationshemmern, Antioxidantien, Färbemitteln, Vitaminen, Mineralsalzen, Geschmacksmodifizierungsmitteln, Glättungs-, Aufbau-, Isolierungsmitteln und deren Gemischen.

6. Zusammensetzung gemäß einem der Ansprüche 2 bis 5, **dadurch gekennzeichnet , dass** sie einen zusätzlichen Wirkstoff oder mehrere zusätzliche Wirkstoffe umfasst.

7. Verwendung von 3,4-Diaminopyridin-Tartrat oder -Phosphat zur Herstellung einer pharmazeutischen Zusammensetzung, die zur Behandlung von Botulismus, Myasthenie, myasthenischen Syndromen oder Müdigkeit in Verbindung mit einer neurologischen Erkrankung, wie multiple Sklerose oder amyotrophe Lateralsklerose, bestimmt ist.
